Europäisches Patentamt

(19) European Patent Office

Office européen des brevets

(11) Publication number: **0 160 389**
**A1**

# (12) EUROPEAN PATENT APPLICATION

(21) Application number: 85302092.3

(22) Date of filing: 26.03.85

(51) Int. Cl.⁴: **G 01 N 33/86**
**C 12 Q 1/56, G 01 N 33/48**

(30) Priority: 27.03.84 US 594027
23.10.84 US 663849

(43) Date of publication of application:
06.11.85 Bulletin 85/45

(84) Designated Contracting States:
AT BE CH DE FR GB IT LI LU NL SE

(71) Applicant: INTERNATIONAL HEALTH SERVICES
1898, Bay Road
East Palo Alto California 94303(US)

(72) Inventor: Stewart, David L.
568 7th Street
Montara California 94018(US)

(72) Inventor: Nguyen, The Thanh
947 Tamarick Lane 1
Sunnyvale California(US)

(72) Inventor: Beal, Charles B.
1312 Bellair Way
Menlo Park California(US)

(74) Representative: Ellis-Jones, Patrick George
Armine et al,
J.A. KEMP & CO. 14 South Square Gray's Inn
London WC1R 5EU(GB)

(54) A method of determining the clotting time of blood and particulate reagents therefor.

(57) A method of determining the clotting time of blood, particularly whole blood, is presented, in which the clotting time is deliberately lengthened, by dilution and spreading, to separate fibrin web formation from clot formation, and the time of fibrin web formation is used as the end point. The fibrin web can be visually enhanced by use of a particulate reagent, particularly of latex particles, and particularly a bidisperse mixture, whereby the latex particles agglutinate with the fibrin strands and make the time of fibrin web formation easier to measure.

EP 0 160 389 A1

A METHOD OF DETERMINING THE CLOTTING TIME OF BLOOD

AND PARTICULATE REAGENTS THEREFOR


This invention relates to improvements in the art of determining blood clotting times.

Blood clotting is the visible result of the conversion of fibrinogen into an insoluble meshwork of fibrin. Fibrinogen is partially digested by thrombin to yield fibrin monomers; these polymerize into strands of fibrin, and the strands are cross-linked by fibrinoligase (which is activated by thrombin) in the final step in one or both of two convergent and interwined chains of chemical reactions, the extrinsic and intrinsic pathways.

Several methods of determining the normality of the blood coagulation mechanism are known. Among the more important tests are "prothrombin time," "activated partial thromboplastin time," and "thrombin time." (See R. Ravel, Clinical Laboratory Medicine 71-73 (4th ed. 1984) for brief descriptions of these tests.) The aforementioned tests are performed on blood plasma or serum rather than on whole blood.

It is recognized that there are difficulties in determining the endpoint of a blood plasma clotting element by visual observation of the formation of the clot (US-A-3,458,287).

These problems are exacerbated when measurement of whole blood clotting time is attempted. As noted in US-A-3,492,096, "even a most painstaking and careful observation sometimes fails to show the existence of the first clot. Being of the same color, and differing in outward physical appearance only in its more viscous nature, the first small clot is not readily distinguishable from the blood itself." The solution, continually tilting a tube having filter means in which the clot would be retained, is not entirely satisfactory. Either the technician must have

- 2 -

one hand on the stopwatch and the other tilting the tube throughout the test, or automatic tube-tilting mechanisms must be provided.

We have found that it is possible to determine clotting time by timing the formation of the fibrin web, and that it is possible to enhance the visibility of this web by use of certain particulate reagents.

It has also been said that it is undesirable to adopt procedures which tend to extend clotting times (see US-A-4,289,408 and 2,921,000). We have found, however, that extension of clotting times more distinctly separates the time of fibrin web formation from the time of clot formation and thus facilitates the determination of clotting time. While US-A-1,614,337 also teaches retardation of coagulation, his purpose was not the measurement of clotting times, but rather the production of fibrin from animal blood without entrained fluid.

Various particles have been utilized as Hageman factor activators, the Hageman factor being a participant in the intrinsic pathway. These includes diatomaceous earth, bentonite, kaolin, sand and silica (see USA-A-3,295,210 and 4,455,377). Latex particles do not significantly activate Hageman's Factor.

An early use of latex particles in a diagnostic test of blood is described in Singer and Plotz. Am. J. Med. 21:88-92 (1956). For a review of latex particle assays, see Hechemy and Michaelson, Laboratory Management, 27-40 (June 1984) and 26-35 (July 1984).

Latex particles have been used in agglutination immunoassays. Here, antigen-antibody binding is irrelevant, the dominant concern being the interconnection of the distinctive latex particles by fibrin strands to form a visually enhanced fibrin web.

Again in agglutination immunoassays, mixtures of latex particles have been used (see, e.g. US-A- 4,191,739; 4,184, 849; 4,115,535; and 4,305,925.) However, these references do not teach the use of a mixture of large and small particles to control the amount and rate of flocculation, and hence the apearance of the agglutinate. Moreover, several references teach against use of mixed particle sizes. US-A-4,421,896 indicates at column 15, line 55 to column 16, line 3 that particles should be monodisperse. US-A-4,321,058 states, at column 4, lines 20-23, that "to increase the reproducibility of the results of measurement, it is preferred to use particles having a relatively narrow range of size distribution."

Charged substances have been used in paracoagulation assays for fibrinogen and derivatives, where they alter the solubility of the fibrinogen and cause its precipitation (see Wilner, Prog. Hemostatis Thromb. 4:211 (1978)).

US-A- 4,429,040 (Becker) describes a method of assaying fibrin monomer in biological fluids wherein the fluid is incubated with latex particles and the agglutination of fibrin monomer to latex determined. It is said that "fibrin monomer, in contradistinction to fibrinogen and other plasma proteins, displays a high affinity towards hydrophobic latex particles." While it is suggested that this method may also be used for indirect determination of fibrinogen (after using an enzyme such as thrombin to convert the fibrinogen into fibrin monomer), the reference does not disclose that latex particles have any utility in measurement of clotting times, there is no recognition that particle diameter is significant in determining the nature of the agglutination (e.g., floccules v.granules), and there is no consideration that a mixture of particle sizes may visually enhance agglutination over and above that obtained with monodisperse particulate reagent. The Specification emphasizes that what is attempted to be determined is fibrin monomer and "soluble fibrin," Col 3. lines 11-19. The term "soluble fibrin" includes fibrin dimers and fibrin-fibrinogen complexes; it does not include, by definition, fibrin polymerized to the point of insolubility. Our invention is based on detection of insoluble fibrin web as an indication of clotting times. Becker does not teach that polymeric fibrin has a high affinity for latex. Becker uses high latex concentrations; we use low latex concentrations. Becker uses a very small amount of thrombin (enough to convert fibrinogen to fibrin monomer, but not enough to convert all the fibrin monomer to polymeric fibrin); we use an excess of thrombin, added directly or otherwise elicited. Becker's incubation times are as long as 5 minutes; ours are 30-60 seconds for normal subjects. Becker's examples deal with plasma specimens; we can utilize whole blood.

According to the present invention there is provided a method of visually determining the clotting time of whole blood comprising

      (a)   diluting the whole blood with a diluent bearing a coagulation agent;

      (b)   increasing the surface area of the resulting solution to reduce the optical density of the solution; and

      (c)   observing the time of fibrin web formation, as well as a method of determining the clotting time of blood which comprises contacting blood with a clotting agent in the presence of a particulate reagent such that the particles are agglutinated in a fibrin web.

By deliberately lengthening the clotting times, percentage error in measurement is reduced, and it becomes possible to utilize as an endpoint the time of fibrin web formation rather than the vaguer time of clot formation. It also becomes simpler to determine clotting times from a whole blood sample, rather from plasma, and to do so as room temperature, without instrumentation.

Preferably latex particles, and particularly mixtures of two sizes of latex particles, are used to visually enhance the fibrin web. The particulate reagent also reduces the influence of blood composition and temperature. The present invention also provides a coagulation reagent comprising a clotting agent and a plurality of negatively charged particles.

The method of this invention is easily practised utilizing a simple and inexpensive device. A preferred such device is described and claimed in our Application No. (N.39057) filed on even date herewith.

In one embodiment, our invention is a novel method for the use of , say, latex particles in the detection and measurement of biological polymerization reactions. It is applied by way of example to the determination of the time of fibrin web formation during blood coagulation. Latex particles are added to blood and consequently blood proteins are attached onto the particles. Addition of a coagulating substance, e.g. thrombin or thromboplastin, causes one of the absorbed proteins (fibrinogen) to form a reaction product (fibrin) which polymerizes into a fibrin web which is more easily detected due to interactions of the attached latex particles. This makes the time of the web formation more easily measured. This time of web formation is easily correlated with the time of actual clot formation. Further, the structure of the latex-fibrin web is a function of fibrinogen concentration and thus permits simultaneous detection of abnormalities in that concentration.

The invention permits measurement of clotting time to be conducted on a whole blood sample, whether or not treated with an anti-coagulant, rather than a plasma sample.

By the addition of particles which increase the surface area of a volume of blood there is an inducement toward coagulation of the blood. The added particles may be in sufficient quantity to have a total surface area beyond the amount of surface area requred for maximum rate of coagulation so induced so that surface area is not a factor in variations of rate of coagulation otherwise initiated and controlled. Said particles may have intended functions in addition to increasing surface area, for example latex for visual enhancement of the reaction, silica particles for platelet activation, or liposomes for coagulation inducement.

The particles become associated with a reactant or product of the (coagulation) reaction; they can be present in sufficient quantity to make said reactant or product more readily detectable or measurable in one or more respects (e.g. time of reaction) and other characteristics of said reaction also detectable or measurable related to different qualities of particle reaction (e.g. flocculation or absorption).

For example, fibrinogen in anticoagulated blood forms into a fibrin web at a time dependent on the concentration of certain clotting factors (e.g. factor VII) when mixed with thromboplastin. A quantity of a particular type of latex particle (e.g. 0.764 micron diameter white polystyrene) can be added, which makes the time of fibrin web formation more visible. The particles become associated with fibrinogen molecules by passive adsorption when introduced into the blood. The white latex becomes readily detectable by aggregation when the fibrinogen polymerizes to fibrin as a result of adding thromboplastin. The time of this latex-mediated and readily detectable event depends on the concentration of factor VII. But the quality of the event differs depending on the concentration of fibrinogen. The fibrin web appears as a heavy flocculation when fibrinogen concentration is normal, as a granular precipitate when of moderate concentration, and as a thin sheet when fibrinogen is very low.

This invention provides a simple method for addition of particles (e.g. latex) to become associated in a test reaction elicited by addition of a substance (e.g. thromboplastin) wherein said particles become associated with components (e.g. fibrinogen) of said reaction (e.g. fibrin formation) in a manner not directed by the added substance (although said particles and substance may or may not be added in association) and in which addition of two or more classes of particles together yields greater enhancement of the reaction than addition of the two classes of particles singly at any concentration. For example, in the prothrombin time the most readily visible result when adding different quantities of 0.277 micron diameter white polystyrene latex particles is the sudden appearance of small white clumps. It is the gradual appearance of larger clumps when using 0.764 micron latex of the same material. An appropriate combination of quantities of the two particles results in the sudden appearance of large clumps, and the magnitude of the reaction is greater than with additions of either of the two classes of particles alone, at any concentration. Thus addition of two or more types of particles with different characteristics (e.g. diameters) to the reaction make said reaction more readily detectable or measurable (facilitation), or less readily detectable or measurable (inhibition) than said reactants or products alone or in the presence of a single type of particle. The use of particles having different characteristics (e.g. diameter) and which facilitate or inhibit the reaction enable certain specified characteristics of said reaction (e.g. time of occurrence) to be more readily detected or measured than in absence of particles or in the presence of a single type of particle, and so that other characteristics of the said reaction (e.g. concentration of a component such as fibrinogen in prothrombin clotting time) may be more readily discernible by quality of the appearance of the latex particles (e.g. flocculation with higher fibrinogen concentrations but a white membrane at lower concentrations).

In some of the literature in this field, little or no attempt is made to distinguish between the fibrin web formation and the clot formation. However, it is clear that this distinction is a valid one, since at very low fibrinogen concentrations, one can observe the web without later observing the clot. By formation of the web, we mean the polymerization of fibrin monomers into polymeric fibrin, which appears, without latex as opaque, white, glistening strands in the periphery of a viewing chamber. As the strands are crosslinked by fibrinoligase, the meshwork becomes denser, the strands coalesce and thicken, and other blood elements, such as platelets and red blood cells are enmeshed, leaving a dark glob that we call the actual clot.

The time from the activation of the clotting process to the observation of the clot is the clotting time. This time interval is easily correlated with, and calculated from, the observed time interval from activation of the clotting process to observation of the fibrin web.

This latency time for fibrin web formation should not be confused with the duration of this formation from first appearance of fibrin strands to completion of the web. The latter may be thought of as the "precision" of the fibrin web timing.

Blood clotting is one species of "coagulation." While it is possible to differentiate among the terms "coagulation", "aggregation," "agglomeration," and "agglutination," we have used these terms interchangeably. "Flocculation" and "granulation" have been used to delineate reactions yielding particular desirable appearances of agglutinate, i.e. to refer to the nature of the agglutination.

Blood dilution retards the clotting reaction and thereby lengthens by a few seconds the interval between the visible formation of the fibrin web and the actual visible clot formation. Preferably, the volume of diluent is at least four times the volume of the sample.

Additionally, spreading the blood makes it easier to visualize the formation of the web. For example, in a device employing the method of this invention, the blood was spread within a viewing chamber 5 x 7 cm. in viewing area and only a few mm thick. (This chamber accommodated a total volume of fluid of 2.5 ml.) The reaction first becomes visible in the pouch around the edges of the thin, fairly clear layer of blood-thromboplastin solution within the viewing chamber.

Together, this dilution and spreading retard normal clotting time to about 60 seconds, as compared to the 10 seconds of the standard test. This reduces the observer's percentage error by a factor of 6. This prolongation also separates the initial formation of the fibrin web formation, a brief physical event, from the actual clot formation by a few seconds. In this manner an observer may time the precise web formation rather than the more protracted formation of the actual clot.

Dilution of the blood also reduces the effective percentage variation in clotting time with the hematocrit. Both dilution and spreading reduce optical density and thus make web formation more visible.

Clotting time is a function of the exposed surface area up to an asymptotic level. It is advantageous to spread the blood over a large surface in a viewing chamber. The addition of latex increases the amount of surface exposed to blood ensuring uniformity of reaction by maximum exposure.

Coloring agents (e.g., stains and dyes) or colored or fluorescent backings may be employed to enhance the visibility of the web.

Also particles may be treated to further enhance detection of the reactions. For example, colored particles can be used and some of these colors (e.g. yellow) contrast with the red blood solution, making their detection easy when aggregated. Also fluorescent particles have been viewed under ultraviolet light; reactions of these particles are even more easily detected, making the detection of very low concentrations of fibrinogen very simple.

A number of substances are capable of inducing blood coagulation. Among those used in blood coagulation measurements are thrombin, thromboplastin, partial thromboplastin, activated partial thromboplastin and derivatives and analogs such as Reptilase -R (Pentapharm Ltd., 4002 Basle, Switzerland), a thrombin-like enzyme isolated from the venom of Bothrops atrox. Any agent which facilitates the clotting of blood along either pathway may be considered a clotting agent as this term is used herein.

The thromboplastin, for example, may be carried to the reaction by the latex particles. Liquid thromboplastin mixed with a latex suspension is easier to measure and handle than each individually or the light, fine powder of lyophilized thromboplastin alone. The lyophilized combination can be used as dry reagent and then dissolved in diluent and added to the blood. Both web formation due to the thromboplastin and the flocculation due to the latex occurs as when the latex is in liquid suspension and the thromboplastin is lyophilized alone.

Lyphilized reagents are known - see US-A-3,918,908, US-A-3,497,319 and US-A- 3,395,210, but these references do not teach lyophilization of latex particles with a clotting agent.

Other reagents can be adhered to the latex particles and added to the reaction in order to assure simultaneous delivery of latex and reagent to a particular point.

Our first observation of latex enhancement of fibrin web formation consisted of mixing specified quantities of a complex collection of different white latex particles with the saline diluent used to lengthen clotting times for measuring prothrombin times. Addition of this particulate reagent to blood in the presence of a clotting agent resulted in white masses forming in blood at a time similar to the time a fibrin web occurred when using the same blood without the latex. The prothrombin time device used 50 mg of thromboplastin, 2.0 ml of diluent, and 0.5 ml of whole blood and the clotting time was about one minute for blood from normal donors. These white masses were more readily visible than the normal fibrin web.

Latex particles in various suspensions were obtained from three different manufacturers. Observations made using particles from one supplier were confirmed using particles from other suppliers. Variations between similar particles from different manufacturers are usually due to differences in suspension media (e.g. presence of surfactant) or packaging (e.g. solids content of the particle suspension).

Initial experiments were conducted to determine if the observation of clumping was due to some form of spontaneous agglutination or was associated with the clotting of the blood. The masses did not form unless a coagulant such as thrombin or thromboplastin was added to the dilute blood. The time of mass formation was associated with the time of coagulant addition rather than latex addition, and the time of massing varied with the clotting time of the blood used. Microscopic observation of the masses indicated close association of the latex particles with fibrin and showed the masses to be a flocculation reaction. Electron micrographs confirmed the microscopic observations.

The time of onset of the reaction with latex present did not differ from expected values for dilution with saline alone. That is, fibrin web formation occurs at about 30 seconds with 1 ml saline and at about 60 seconds with 2 ml saline dilution, when using a device employing the method of this invention at room temperature. No significant change in the time of fibrin web formation to the presence of the particles other than as a simple increased dilution of the blood is observed. Thus, the role of the particles is to visually enhance the web, not to alter its time of onset.

Two types of experiments indicated that, prior to coagulation, passive adsorption of materials circulating in blood is of greater importance than chemical bonding for clotting time tests (although chemical bonding may also be used and may be useful in other ways in this test and in other tests). Latex particle suspensions which included surfactant were not as efficient as particle suspensions supplied without surfactant. When small quantities of surfactants, which coat hydrophobic surfaces, were added to the prothrombin time formulation, flocculation did not occur although the fibrin web was observed. For example, a threshold for observation of latex enhancement was obtained by adding 50 µl of 0.711µ diameter, 8.7% solids, polystyrene latex particles in a surfactant-free saline suspension to a prothrombin time formula consisting of 0.5 ml normal whole blood, 50 mg of thromboplastin, and 1.0 ml of diluent which resulted in fibrin web formation in 30 seconds. this is the prothrombin time formulation used for all subsequent experiments described herein with variations in latex particles unless otherwise specified. Addition of 20 to 40 micro moles of the surfactant TWEEN 20 prevented the latex enhancement but not the fibrin web formation.

Another set of observations indicated the importance of passive adsorption over chemical bonding during the initial mixing of the particles. Particles formed of polystyrene generally have sulfate functional end groups available for chemical bonding. Particles made of other polymers may have other functional end groups with hydroxyl, carboxyl and amine being commonly available. The concentration of latex necessary to obtain a given level of enhancement depends primarily on particle diameter and solids content rather than the functional end group of the particle used. However, for particular applications, use of a particular end group may be desirable. We have a slight preference for amine end groups.

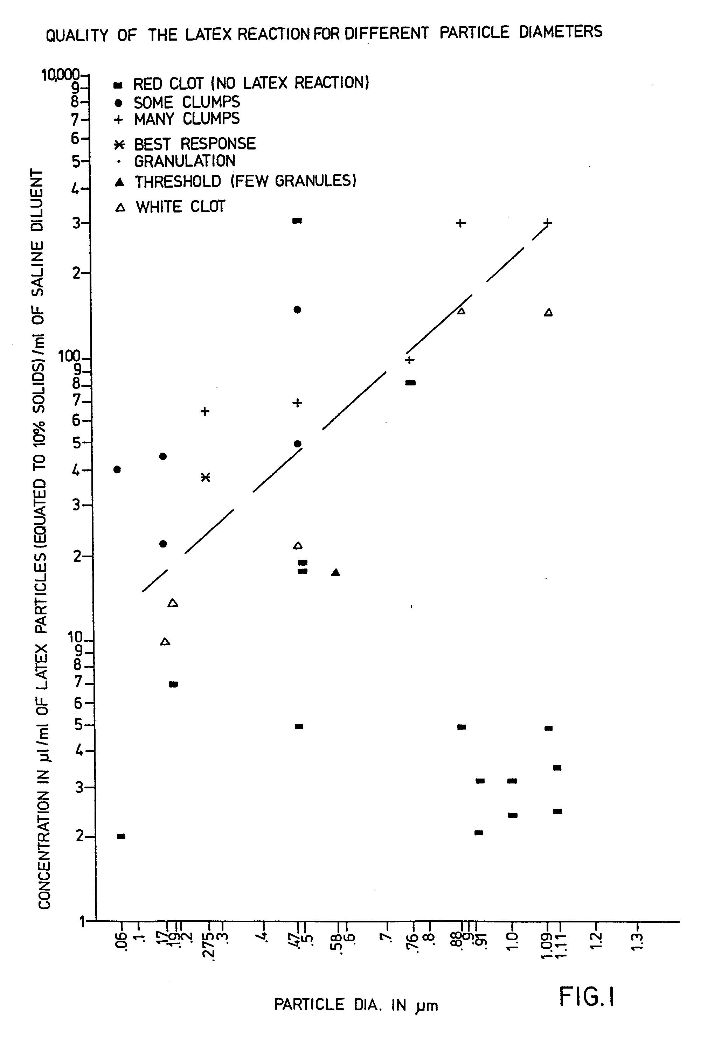

The quality of the observed latex enhancement of the clotting time test was found to vary systematically with concentration and diameter of particles. Particles less than 0.1 microns in diameter produced reactions which were difficult to observe with the unaided eye. (Such particles might be useful, however, if observed aided by other means such as fluorescence or magnetism.) Particles greater than 3.0 microns had a poor surface area-to-volume ratio and therefore large quantities of these expensive particles were needed to achieve the desired total surface area. Particles with diameters in the 0.1 to 3.0 micron range were arguably easy to use and observe. For any particular diameter an optimal particle concentration can be determined without undue experimentation. Either increases or decreases from the optimal particle concentration changed the quality of the reaction, generally in the following sequence: (1) less than maximum amount of flocculation, (2) few floccules with numerous small granules, (3) numerous granules only, (4) granules and a white coating of the clot, (5) a white coated clot only, (6) a small white fibrin membrane, separate from the clot and (7) a non-enhanced fibrin web. The quantity of particles of different diameters was varied systematically and the quality of the reaction noted. Different quantities for different diameters were required to yield comparable results as shown in Figure 1.

Reference should be made to the accompanying drawings which are included merely by way of example and in which

Figure 1 shows the relationship between particle size, particle suspension volume, and quality of agglutination for a monodisperse latex suspension/clotting agent addition.

Figure 2 shows the relationship between the quality of agglutination to relative concentrations of 0.277 and 0.764 micron latex particles in bidisperse latex suspension/ clotting agent addition.

The total particle surface area per volume of latex particle suspension may be calculated from the solids content, the particle density, and the particle diameter. When this was done for suspensions of particles of different diameters, it was found that a single total surface area yielded each quality of latex enhancement (for a given volume of sample, dilution ratio, and fibrinogen concentration).

The determination that a particular surface area of latex particles is associated with a particular quality of visual enhancement of the clotting time is consistent with the observation that the initial association of latex with the reaction is mediated by passive adsorption. It is known that adsorption of a ligand onto latex particles is a function of the available surface area and ligand concentration. In our case, the fibrinogen molecule

- 14 -

apparently adsorbs to an optimal number of available spaces on an optimal quantity of latex when the total latex surfaces area is about 17-18 $cm^2$, the volume of the diluent and blood is as given previously, and the concentration of fibrinogen is at a normal level. The quality of visual enhancement changes with changes in fibrinogen concentration. If fibrinogen is at a normal level, then it will be optimally adsorbed and the amount of flocculation maximal. If the fibrinogen concentration is less than normal, less will be adsorbed and granules or a white sheet will form. The time of occurrence of the clot after addition of tissue factor as thromboplastin is a function of circulating clotting factors other than fibrinogen. The concentration of fibrinogen is qualitatively indicated by the structural appearance of the latex agglutination.

The fibrinogen-latex coupling is not macroscopically visible. Fibrinogen cleaves and polymerizes to form fibrin at a specific time after the addition of thromboplastin. It is the polymerization into a web which is visually enhanced by the associated latex particles. Thus, it is the addition of thromboplastin or other clotting agent which determines the time of appearance of agglutinated particles joined to fibrin molecules. This agglutination reaction is secondary to the passive adsorption of fibrinogen to the latex particles, but occurs without any additional treatment of the blood or the particles. That is, the blood coagulation test is a two stage latex reaction performed by a one-step latex addition.

Although many molecules have affinities for latex, agglutination assays typically employ pure coating agents. This method does not require pure coatings of fibrinogen on latex, rather, it permits competitive absorption of blood proteins, including fibrinogen, on the latex particles, the conversion of fibrinogen to fibrin and the polymerization of the latter being responsible for the desired agglutination. Variations in the structure of the aggregated mass are micro- and macroscopically visible and closely correlated with initial concentration levels of the precursor protein (fibrinogen) in the elicited reaction (fibrin formation).

In the blood coagulation tests, the dilute blood is a pinkish homogeneous fluid when viewed macroscopically. Addition of a coagulating substance (e.g. thromboplastin) elicits fibrin formation at a time predetermined by several factors and variables. A quantity of latex particles can be added so that a maximum degree of flocculation occurs if fibrinogen concentration is at a standard level (e.g. 4.0 mg/ml). The floccules appear as large white clumps in a reddish fluid. The degree of flocculation decreases as the fibrinogen concentration varies either above or below the standard level. The magnitude of the flocculation decreases so that numerous white granules appear instead of clumps when fibrogen concentration differs either above or below the standard level. However when fibrinogen concentration is significantly high (e.g. 7.0 mg/ml) or low (e.g. 1.0 mg/ml) then no type of flocculation occurs; rather the latex particles aggregate with the fibrin web formed about the blood clot, giving the clot the appearance of a white coat.

These structural variations might be used to qualitatively measure the concentration of other specified proteins, which, after addition of a reactant, polymerize or otherwise react to cause latex agglutination.

Some mixtures of particle diameters were found to be more efficacious than monodisperse particles in producing aggregation. Subsequent experiments have shown that specific mixtures facilitate particular reactions, and that other specific mixtures inhibit particular reactions, and that the different interactions of particle diameter mixtures are useful for diagnostic purposes.

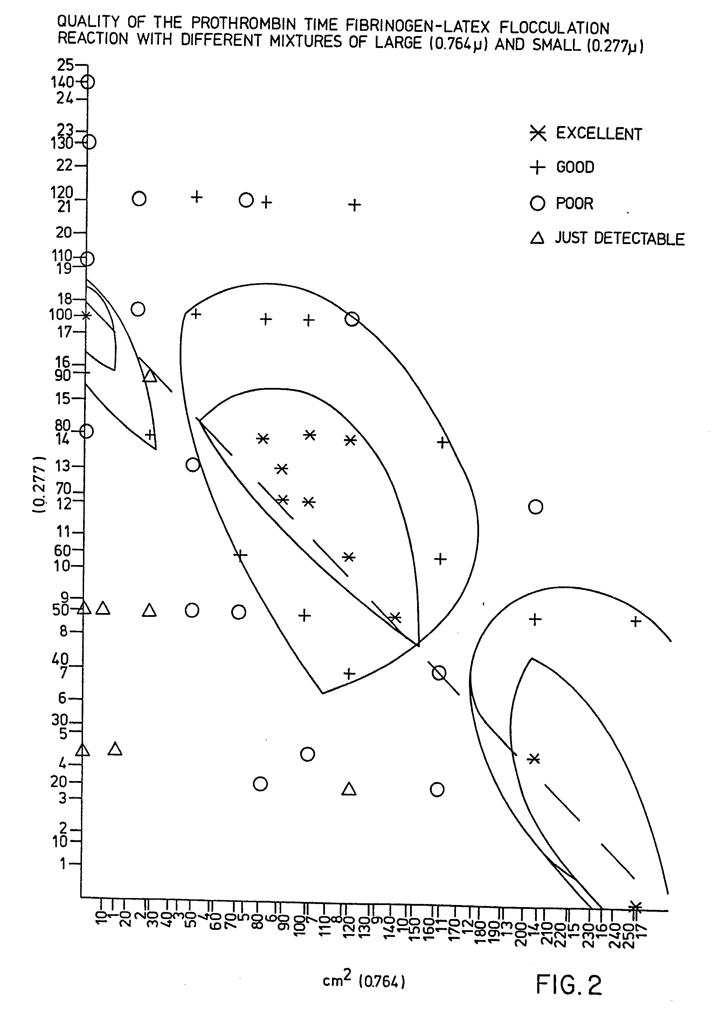

A compilation of several experiments is illustrated in Figure 2. Quantities of two latex particles were used, which differed in diameter only; 0.277 microns and 0.764 microns, these being white polystyrene latex particles in saline (8.1% and 8.7% solids, respectively) suspension (Interfacial Dynamics Corporation). Each axis of the figure shows both the liquid volume of the particle suspension and the corresponding total surface area of the particles in that column of suspension for each of the particle diameters used. The quantities of each particle indicated in the figure was added to one ml of saline, which was then mixed with 50 mg of lyophilized Thromboplastin and calcium (Dade). That mixture was added to 0.5 ml of anticoagulated (3.8% sodium citrate) whole blood. Both the time (about 30 seconds) and quality of fibrin-latex aggregations were observed for each mixture.

A volume and corresponding total surface area for each particle diameter was determined to be optimal for flocculation production (e.g. 0.277:100 µl, 17-18 $cm^2$; 0.764;250 µl, 17-18 $cm^2$).

Values above or below the optimum yielded qualities of aggregation different from maximum flocculation (i.e. small clumps, granules, white coated clot, white membrane, or non-enhanced fibrin web), as more fully described in connection with Figure 1. The quality of the aggregation was determined for the total surface area of the particles added when adding particles of a single diameter. The surface area necessary to yield maximum flocculation was always about 17-18 $cm^2$, under the aforementioned standard conditions with slight variations of level of maximum flocculation with particle diameters.

The total surface area necessary to yield maximum flocculation, or other selected qualities of aggregation, differed with different mixtures of particle diameter. The dashed line in Figure 2 shows the combination of particle diameter mixtures yielding a constant total surface area of 17 $cm^2$.

Flocculation reactions are detectable over a much greater range of total particle surface areas when using certain combinations of particle diameters than when using particles of a single diameter. For example, when using 0.277 micron diameter particles alone, maximum flocculation occurs with about 17 $cm^2$ of surface area; this decreases significantly in magnitude with an increase or decrease of one $cm^2$ surface area, and becomes poor after an increase or decrease of another $cm^2$ of surface area. In contrast, if about 10 $cm^2$ surface area composed of 0.764 micron diameter particles is used consistently, and different amounts of 0.277 diameter particles are added, then good flocculations occur with total combined particle surface areas as low as 14 $cm^2$ (10 + 4) and up to 25 $cm^2$ (10 + 15) or greater. The value of this observation lies in easy detection and measurement of clotting times over a wide range of fibrinogen concentrations.

Other mixtures of particle sizes decrease the range of total surface area sufficient to yield a clear flocculation. For example, if the number of 0.764 μ diameter particles is kept constant at a total surface area of 2 $cm^2$, and the number of 0.277 μ diameter particles is varied, then a clear flocculation only occurs with a total surface area of about 14 $cm^2$ (no flocculation at 18 $cm^2$ surface area). The value of this finding is in detecting a narrow range of fibrinogen concentrations.

Thus, a range of fibrinogen (or other protein) concentrations of diagnostic significance may be selected to be detected by predetermining a mixture of particle diameters and their relative concentrations.

There is also a total surface area for mixtures of particles of different diameter at which the quality of the reaction alters. In the clotting time reaction there is a minimum total surface area of particles necessary for a white-coated clot to appear and below which only a more difficult to visualize membrane is detectable. The value of this finding is that different concentrations of fibrinogen may be determined using a preselected mixture of particles of different diameters more accurately than when using particles of a single diameter.

Another series of observations are illustrated in Figure 2. A group of "excellent" reactions are shown to have occurred for certain combinations of particle diameter quantities. These excellent reactions appeared to result from facilitation of the best features of the best reactions observed when using single diameters of particles alone. The clearest reaction observed using 0.764 micron diameter particles alone was a gradual (1-2 second duration) development of large clumps or floccules while the clearest reaction observed using 0.277 micron diameter particles alone was the sudden appearance of fewer and smaller floccules. The excellent reactions consisted of very suddenly appearing large floccules occurring more rapidly and in greater number than with particles of any single diameter. The significance of this is that combinations of particles of different diameters may be used to yield a more obvious and measurable reaction. For example, the great majority of individuals have fibrinogen concentrations which fall within the "normal" range. A selected mixture of large and small (e.g. 100 µl of 0.764 micron and 75 µl of 0.277 micron diameter) particles yielded an exceptionally obvious agglutination reaction, sufficiently great for a novice to detect and measure. Any difficulty in detection would indicate an abnormal fibrinogen level. Ease of detection and measurement of other substances may be improved and particles other than latex may be used similarly.

Another benefit of the use of mixtures is reduced cost. Latex is sold by liquid volume. Larger particles are sometimes more expensive than smaller ones and are preferred because their aggregation is more obvious. However, a larger quantity is necessary to attain a selected total surface area when using larger particles. Adding smaller volumes of smaller particles decreases the total cost of particles.

It can thus be seen that by appropriate selection of the particle size(s) and concentration(s) it is possible to provide a desired quality of agglutination eg. maximum flocculation or maximum granulation, over a desired range of fibrin concentrations, to minimise the duration of the fibrin web formation, to maximise the visibility of the fibrin web.

While this invention is not limited by the following postulation of a mechanism for the latex particle enhancement observed, the explanation may nonetheless prove helpful in selecting the best particulate reagent for a particular application.

The mechanism of the facilitatory and inhibitory interactions among mixtures of particles of different diameters is believed to be related to the electrical charge of the particles. Latex particles have a negative charge directly related to their particle diameter. The particles in suspension maintain an average distance from one another by forces of mutual repulsion of like charges and surface attractions. These particles may be joined together to form aggregates by molecules of sufficient length to bridge the distance between the particles and which are attracted to the particle surfaces. Many molecules are attracted to latex in blood, but none have all the characteristics necessary to yield detectable aggregation. Fibrinogen is one of the molecules attracted to latex surfaces but is not capable of bridging the distances between particles. However, when thrombin, or a substance which yields or acts in a manner similar to thrombin (eg. thromboplastin), is added it cleaves peptides from fibrinogen, yielding monomeric fibrin, which then polymerize. The longer molecular chains bridge the spaces between particles allowing their aggregations. Larger particles maintain greater distance between themselves, requiring greater lengths of fibrin to be generated before aggregating. Once they form, however, the aggregations are larger and more easily detected than is the case when using smaller particles. The use of a selected combination of larger and smaller particles allows the average distance between particles to be made smaller, which allows the initially formed shorter fibrin molecules to bridge the gaps and form aggregates which are more easily detected due to the inclusion of larger particles. In order for an aggregation to occur, a sufficient amount of fibrinogen must become adsorbed to a sufficient number of particles prior to its polymerization to fibrin. This is a function of the total available surface area and the concentration of fibrinogen. However, the two particle sizes in part react independently during polymerization. When the available surface area of one of the particles is small the magnitude of the aggregation is a function of the surface area of the particle with the larger available surface area (dominant) as if the available fibrinogen became selectively attached to those particles, which contribute more of the available surface area. There is an area of relative concentrations of particles where a sufficient amount of fibrinogen has been adsorbed to the non-dominant particle, so that the dominant one (with the greater surface area) has attracted only sufficient fibrinogen to yield a weak aggregation upon polymerization. There is also an area of relative concentrations of particles such that the available fibrinogen is adsorbed to the available surface area and the concentration of fibrinogen per particle advantageously corresponds to the distances between particles so that a greater total surface area may be aggregated (facilitation).

Electron micrograph images of a dominant population of small particles (100 ml) and a weak population of larger particles (25 ml) show that the reaction is predominantly of very short fibrils rather than longer strands.

The postulated mechanism is significant in that one may select particle diameter mixtures so as to maintain them a pre-selected average distance from one another so that agglutination will occur if a ligand of a particular length is or becomes available to bridge gaps between particles.

Because of the importance of "charge" in the postulated mechanism, it is believed that other charged particles may prove useful in these determinations. Latex particles are, however, preferred.

A number of theoretical studies of the stability of latex dispersions have been published. Ottewill and Shaw, Discussions of the Faraday Society, 154-163 (1966), discuss the stability of monodisperse polystyrene particles. On both theoretical and experimental grounds, it concluded that the flocculation concentration was independent of particle radius, or at most, only weakly dependent (159-160). Examing the mutual coagulation of particles of two sizes and opposite charges, Hogg, et al., Id., 62:1638-1651 (June 1966), concluded that the effect of small differences in particle size on the variation of stability with pH, could be safely ignored.

Wise and Healy postulate, Id., 66:490-499 (1970), that, for monodisperse systems, the stability of the dispersion first rises and then falls with particle radius. They also discuss certain bidisperse systems.

Of course, these theoretical analyses are directed at interactions between uncoated particles. Interactions between coatings will obviously affect the stability of the dispersion.

Two or more particles may be added to a reaction so that each reacts with a different component or resultant of a reaction so that the reaction may be more readily detected and or measured and the different components or resultants more easily detected or measured than in absence of said particles.

Different reagents can be coupled to particles of different diameters. Substances (e.g. peroxidase, gold, biotin, dextran, etc.) may be attached to a particle used for enhanced ease of detection or measurement, and these substances may combine with targeted materials in the test solution, or with reagent material mediating a reaction to be detected, or with resultants of a reaction occurring in the test solution and said substances themselves further enhancing ease of detection or measurement. Two or more of said other substances may be attached to one, or more than one, type of particle for further enhancement of ease of detection or measurement.

Other apparatus can be used to adsorb substances and then collect by attraction (e.g. magnetically) the particles to be treated for presence of said substance, by introduction of a reagent.

Other proteins and other materials which can be made to adsorb to or combine with latex particles and which can be made to react with one another may be tested for concentration in the same way as fibrinogen concentration may be detected and measured as described above. In particular, where the reaction product is of considerably different molecular length from that of the substrate, it may bridge the gap between particles, as does the fibrin polymer. Thus, the particulate reagents of this invention· may find particular use in enhancing polymerization or dimerization reactions. While latex particulate reagents have been described herein to enhance the visibility of the agglutinate formed by the polymerization of fibrin, they may also be of value in enhancing the visibility of other polymerization or oligomerization products. For example, it might be used in assays of actin polymerization, – see Pollard, Polymerization of ADF-Actin, J. Cell Biol. 99:769-777 (September 1984). Also, particles other than latex (e.g. silicas, cells, erythrocytes, or liposomes) may be used in a similar fashion or in conjunction with latex.

While this invention has particular applicability to whole blood testing, with suitable modifications it may be

- 23 -

utilized with specimens of blood plasma. Where not inconsistent with the context, or otherwise specified, the term "blood" is inclusive of blood plasma as well as whole blood.

## CLAIMS

1. A method of visually determining the clotting time of whole blood comprising

(a) diluting the whole blood with a diluent bearing a coagulation agent;

(b) increasing the surface area of the resulting solution to reduce the optical density of the solution; and

(c) observing the time of fibrin web formation.

2. A method according to claim 1, in which, prior to step (c), the blood is treated with a coloring means which enhances the visibility of the fibrin web.

3. A method according to claim 1 or 2, in which the fibrin web formation is observed against a contrasting background.

4. A method according to any one of claims 1 to 3, in which the coagulation reagent comprises a clotting agent and a particulate reagent.

5. A method of determining the clotting time of blood which comprises contacting blood with a clotting agent in the presence of a particulate reagent such that the particles are agglutinated in a fibrin web.

6. A method according to claim 4 or 5, in which the particulate reagent comprises a plurality of latex particles.

7. A method according to claim 6 in which the latex particles are of polystyrene.

8. A method according to any one of claims 4 to 7, in which the mean diameter of the particles is from 0.1 to 3.0 microns.

9. A method according to any one of claims 4 to 8 in which the particles are of at least two different sizes.

10. A method according to claim 9 in which the particles are of size 0.764 microns and 0.277 microns, the ratio between the two sizes falling within the "good" or "excellent" regions of Figure 2 of the accompanying drawings.

11. A method according to claim 10 in which the said ratio between the two sizes falls within the "excellent" region of Figure 2.

12. A method according to any one of claims 4 to 11 which comprises contacting the blood sample with a volume of a latex particle suspension, said particles having a diameter D in microns related to said volume in microliters which is less than that represented by the dashed line on Figure 1 of the accompanying drawing, with adjustment for dilution ratio and solids content, and a clotting agent.

13. A method according to any one of claims 4 to 12 in which the particles are treated to enhance their detection.

14. A method according to any one of claims 4 to 13 in which the clotting agent is thrombin, thromboplastin, partial thromboplastin or activated partial thromboplastin.

15. A method according to any one of the preceding claims in which at least four times the volume of the blood is added to the blood.

16. A method according to any one of the preceding claims in which an aggregation inhibitor or facilitator is added.

17. A method according to any one of the preceding claims for measuring blood clotting time and simultaneously detecting abnormal fibrinogen concentration in blood, comprising mixing a sample of blood with a latex particulate reagent of predetermined particle sizes and concentrations in the presence of a clotting agent and obseving both the time to onset of fibrin web formation and the quality of the agglutination of the latex particles and the fibrin, said sizes and concentrations having been selected to yield a flocculate when the fibrinogen concentration is normal.

18. A coagulation reagent comprising a clotting agent and a plurality of negatively charged particles.

19. A reagent according to claim 18, in which the particles are latex particles, liposomes or erythrocytes.

20. A reagent according to claim 18 or 19 in lyophilized form.

21. A reagent according to any one of claims 18 to 20 wherein said particles have been treated to facilitate or inhibit the coagulation response of a substance with which the reagent is contacted.

22. A reagent according to any one of claims 18 to 21 which has one or more of the features of claims 6 to 11, 13 and 14.

23. A method of determining the time for a monomer to polymerize or oligomerize, which comprises mixing the monomer with dispersed particles in the presence of a polymerization agent and measuring the time elapsing until an aggregation of the particles with the resulting polymer or oligomer is observed.

24. A method according to claim 23 in which the monomer is fibrin monomer or actin.

25. A method according to claim 23 or 24 in which the particles have one or more of the features of claims 6 to 11, 13 and 18.

QUALITY OF THE LATEX REACTION FOR DIFFERENT PARTICLE DIAMETERS

Legend:
- ■ RED CLOT (NO LATEX REACTION)
- ● SOME CLUMPS
- + MANY CLUMPS
- ✳ BEST RESPONSE
- · GRANULATION
- ▲ THRESHOLD (FEW GRANULES)
- △ WHITE CLOT

Y-axis: CONCENTRATION IN µl/ml OF LATEX PARTICLES (EQUATED TO 10% SOLIDS)/ml OF SALINE DILUENT

X-axis: PARTICLE DIA. IN µm

FIG.1

QUALITY OF THE PROTHROMBIN TIME FIBRINOGEN-LATEX FLOCCULATION
REACTION WITH DIFFERENT MIXTURES OF LARGE (0.764μ) AND SMALL (0.277μ)

✻ EXCELLENT

+ GOOD

○ POOR

△ JUST DETECTABLE

cm² (0.764)

FIG.2

## EUROPEAN SEARCH REPORT

**European Patent Office**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X Y | EP-A-0 039 885 (BOEHRINGER MANNHEIM GmbH) * Whole document * --- | 1-25 | G 01 N 33/86 C 12 Q 1/56 G 01 N 33/48 |
| D,Y | WO-A-8 304 105 (COOPER LABORATORIES INC.) * Abstract; pages 1-3 * --- | 14,16 | |
| P,Y | EP-A-0 123 883 (BEHRINGWERKE AG) * Abstract; pages 1-5 * --- | 14,16 | |
| Y | EP-A-0 038 181 (TECHNICON INSTRUMENTS CORP.) * Abstract; claim 7; example 2 * --- | 6-10 | |
| Y | GB-A-2 123 146 (ABBOTT LABORATORIES) * Abstract; page 1, lines 112-128 * --- | 6-10 | **TECHNICAL FIELDS SEARCHED (Int. Cl.4)** |
| Y | CHEMICAL ABSTRACTS, vol. 82, no. 3, 20th January 1975, page 213, no. 13297v, Columbus, Ohio, US; U.P. MUELLER et al.: "Sensitive latex agglutination method for detecting soluble fibrins in plasma" & SCHWEIZ. MED. WOCHENSCHR. 1974, 104(39), 1391-1392 * Abstract * --- -/- | 1 | G 01 N C 12 Q |

The present search report has been drawn up for all claims

| Place of search | Date of completion of the search | Examiner |
|---|---|---|
| THE HAGUE | 01-07-1985 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82

**European Patent Office**

**EUROPEAN SEARCH REPORT**

| DOCUMENTS CONSIDERED TO BE RELEVANT | | | Page 2 | |
|---|---|---|---|---|
| Category | Citation of document with indication, where appropriate, of relevant passages | | Relevant to claim | CLASSIFICATION OF THE APPLICATION (Int. Cl.4) |
| Y | BIOLOGICAL ABSTRACTS, vol. 75, no. 5, May 1983, no. 34744; D.L. DUNN et al.: "Fibrin in peritonitis: 3 Mechanism of bacterial trapping by polymerizing fibrin" & SURGERY (ST LOUIS) 92(3), 513-519 * Abstract * --- | | 1 | |
| Y | EP-A-0 019 741 (BEHRINGWERKE AG) * Abstract; claims 1,2 * --- | | 6-10 | |
| P,A | EP-A-0 118 894 (E.I. DU PONT DE NEMOURS AND COMPANY) --- | | | |
| A | US-A-4 419 453 (L.C. DORMAN et al.) --- ----- | | | TECHNICAL FIELDS SEARCHED (Int. Cl.4) |

The present search report has been drawn up for all claims

| Place of search THE HAGUE | Date of completion of the search 01-07-1985 | Examiner OSBORNE H.H. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82